(19) Europäisches Patentamt
European Patent Office
Office européen des brevets

(11) EP 4 551 202 B1

(12) EUROPEAN PATENT SPECIFICATION

(45) Date of publication and mention of the grant of the patent:
**10.06.2026 Bulletin 2026/24**

(21) Application number: **23738761.8**

(22) Date of filing: **06.07.2023**

(51) International Patent Classification (IPC):
***A61K 31/122*** (2006.01) ***A61K 31/136*** (2006.01)
***A61K 31/137*** (2006.01) ***A61P 17/00*** (2006.01)

(52) Cooperative Patent Classification (CPC):
**A61K 31/122; A61K 31/136; A61K 31/137; A61P 17/00**

(86) International application number:
**PCT/EP2023/068695**

(87) International publication number:
**WO 2024/008861 (11.01.2024 Gazette 2024/02)**

(54) **NAPHTHAZARIN DERIVATIVES FOR TREATMENT OF ONYCHOMYCOSIS**

NAPHTHAZARIN DERIVATE ZUR BEHANDLUNG VON ONYCHOMYKOSEN

DÉRIVÉS DE NAPHTHAZARINE POUR LE TRAITEMENT DE L’ONYCHOMYCOSE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **07.07.2022 EP 22183652**

(43) Date of publication of application:
**14.05.2025 Bulletin 2025/20**

(73) Proprietors:
- **Christian-Albrechts-Universität zu Kiel**
  **24118 Kiel (DE)**
- **Helmholtz-Zentrum für Ozeanforschung Kiel (GEOMAR)**
  **24148 Kiel (DE)**

(72) Inventors:
- **PEIFER, Christian**
  **24118 Kiel (DE)**
- **JANSEN, Björn**
  **24118 Kiel (DE)**
- **BAIER, Joana**
  **24118 Kiel (DE)**
- **TASDEMIR, Deniz**
  **24106 Kiel (DE)**
- **WENZEL-STORJOHANN, Arlette**
  **24106 Kiel (DE)**

(74) Representative: **Taruttis, Stefan Georg**
**TARUTTIS Patentanwaltskanzlei**
**Aegidientorplatz 2b**
**30159 Hannover (DE)**

(56) References cited:
- **DESSOLIN J ET AL: "BROMINATION STUDIES OF THE 2,3-DIMETHYLNAPHTHAZARIN CORE ALLOWING EASY ACCESS TO NAPHTHAZARIN DERIVATIVES", THE JOURNAL OF ORGANIC CHEMISTRY, AMERICAN CHEMICAL SOCIETY, vol. 66, no. 16, 10 August 2001 (2001-08-10), pages 5616 - 5619, XP001102735, ISSN: 0022-3263, DOI: 10.1021/JO010137N**
- **SINGH R ET AL: "synthesis and fungitoxicity of some substituted naphthoquinones and heteroarylquinones", INDIAN JOURNAL OF CHEMISTRY, vol. 28, no. 6, 1 January 1989 (1989-01-01), DE, pages 490 - 493, XP093005457, ISSN: 0376-4699**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DE MENEZES HENRIQUE DANTAS ET AL: "Photodynamic treatment with phenothiazinium photosensitizers kills both ungerminated and germinated microconidia of the pathogenic fungiFusarium oxysporum,Fusarium moniliformeandFusarium solani", JOURNAL OF PHOTOCHEMISTRY AND PHOTOBIOLOGY B: BIOLOGY, ELSEVIER SCIENCE S.A., BASEL, CH, vol. 164, 13 September 2016 (2016-09-13), pages 1 - 12, XP029775291, ISSN: 1011-1344, DOI: 10.1016/J.JPHOTOBIOL.2016.09.008
- GUPTA A.K. ET AL: "Onychomycosis: a review", vol. 34, no. 9, 5 June 2020 (2020-06-05), NL, pages 1972 - 1990, XP055874159, ISSN: 0926-9959, Retrieved from the Internet <URL:https://onlinelibrary.wiley.com/doi/full-xml/10.1111/jdv.16394> DOI: 10.1111/jdv.16394
- ZHANG ZIXUAN ET AL: "Shikonin induces necroptosis by reactive oxygen species activation in nasopharyngeal carcinoma cell line CNE-2Z", JOURNAL OF BIOENERGETICS AND BIOMEMBRANES, PLENUM PUBLISHING, NEW YORK, NY, US, vol. 49, no. 3, 25 May 2017 (2017-05-25), pages 265 - 272, XP036799837, ISSN: 0145-479X, [retrieved on 20170525], DOI: 10.1007/S10863-017-9714-Z

## Description

The present invention relates to pharmaceutical compounds for use in the treatment of onychomycoses, which compounds have the advantage of good penetration into keratin, e.g. into fingernails and toenails of humans, and into hooves of animals. Further, the pharmaceutical compounds have the advantage of effectively inactivating both vegetative cells and spores of mycosis that is present within the keratin of fingernails, toenails or hooves.

A further advantage of the pharmaceutical compounds of the invention is that they show little or no penetration into the nail matrix underlying the nail or hoof, so that the anti-mycotic activity of the compounds is generally limited to the nail or hoof.

The pharmaceutical compounds are especially suitable for use in photodynamic treatment of onychomycosis, the therapy or medical treatment of onychomycoses comprising the steps of applying at least one of the compounds onto a nail or hoof, the compound migrating into the nail or hoof, and irradiating the nail or hoof. Under irradiation, the pharmaceutical compounds within the nail or hoof generate reactive oxygen species (ROS), which effectively inactivate both vegetative cells and spores within the nail or hoof. Accordingly, the pharmaceutical compounds have the advantage of fast inactivation of the onychomycosis. Due to the generation of ROS under irradiation, the compounds of the invention can also be designated as photosensitizers.

Onychomycoses are e.g. caused by Trichophyton rubrum and/or Candida albicans.

### State of the art

Pharmaceutical compounds for use in mycosis, e.g. clotrimazole,terbinafin, amorolfin, ciclopirox or bifonazole selectively inhibit the pathway of ergosterol synthesis, which is specific for mycosis but does not occur in humans.

A disadvantage of pharmaceutical compounds used in antimycotic therapy is that only vegetative cells of the mycosis are attacked, allowing spores to persist. Further, such compounds often have low penetration into a nail, resulting in low efficacy of the treatment of onychomycosis, and the necessity for daily application for several months, with high relapse rates.

EP 200219 B1 describes processes for preparing derivatives of naphthazarin.

Papageorgiou et al., Angew. Chem Int. Ed. 270-300 (1999) gives an overview of naphthazarin compounds and their pharmaceutical uses.

Menezes et al., Journal of Photochemistry & Photobiology B 2016, 1 - 12 describes the effect of methylene blue, toluidine blue O and methylene blue N-zinc chloride under irradiation against ungerminated and germinated microconidia of plant pathogenic fungi of the genus Fusarium.

Gupta et al., JEADV 2020, 1972-1990 gives an overview of current treatments of onychomycosis.

### Object of the invention

It is an object of the invention to provide pharmaceutical compounds for use in the treatment of onychomycoses that allow for inactivation of both vegetative cells and spores of the mycosis. A further object is to provide pharmaceutical compounds that have high penetration rates into nail and hooves. A preferred object is to provide pharmaceutical compounds that allow for fast inactivation of onychomycosis, more preferably without impairing the nail matrix of the patient.

### Description of the invention

The invention achieves the object by the features of the claims, especially by providing a pharmaceutical compound for use in medical treatment, e.g. in the medical treatment of fungal infections, preferably for use in the treatment of onychomycoses, including use in the treatment of onychomycoses by spore-forming microorganisms, e.g. by spore-forming fungi, especially in photodynamic treatment, preferably comprising the step of irradiation at a wavelength of 450 to 700 nm, e.g. 500 to 600 nm, preferably at 510 to 530 nm, e.g. at 519 nm, after topical application of the compound to the nail, optionally with a subsequent time period for allowing the compound to migrate into the nail, the pharmaceutical compound the compound as defined in claim 1.

structure

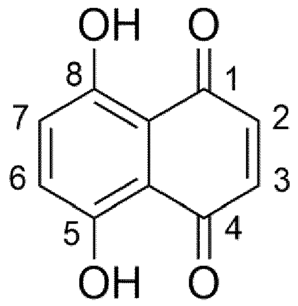

wherein C2 is substituted with R1 and
C3 is substituted with R2 as defined in claim 1,
and the other C-atoms are substituted with H. This structure shows the naphthazarin basic framework.

Optionally, in this structure C2 is substituted with R1, e.g. selected from Table 1, and each of C3, C6 and C7 are substituted with hydrogen.

It was found that the wavelength that is absorbed by the pharmaceutical compound, as well as the migration of the compound into nail or hoof, and/or preferably the metabolic stability of the compound can be varied by the length and degree of saturation of the hydrocarbon group forming R1 and R2, and by its substitution with a halogen atom.

The compounds of the invention have the advantage of generating ROS under irradiation at 450 to 700 nm, e.g. 515 ± 20 nm, preferably 515 ± 10 nm, which is a wavelength for which nail or hoof is sufficiently transmissible. Accordingly, these pharmaceutical compounds are suitable for use in the treatment of onychomycoses as photodynamic treatment, also referred to as photodynamic therapy, by irradiation is effective in generating ROS in those regions within the nail or hoof in which the pharmaceutical compound is present. Preferably, subsequent to topical application of the compound to a nail or hoof, the treatment comprises the step of removing the compound from skin and/or covering skin surrounding the nail to which the compound has been applied topically with a material that is not permissive to the irradiation applied to the nail. Removing the compound from skin and/or covering the skin to which the compound may have been applied is preferred for preventing irradiation, e.g. of 450 to 700 nm, applied to the nail and ROS generated by the compound to affect the skin.

Further, the invention relates to a process for producing the compound for use in the treatment of onychomycoses, and to use of the compound for producing a pharmaceutic composition for treatment of onychomycoses, wherein the treatment is photodynamic treatment, preferably comprising irradiation of the compound following its application to a nail or hoof.

The following table shows specific pharmaceutical compounds of the invention and their $IC_{50}$ values, upper value without irradiation, lower value with irradiation, as determined in in vitro assays.

| name | structure | Ca [nM]<br>$IC_{50}$ without irradiation<br>$IC_{50}$ with irradiation | Tru [nM]<br>$IC_{50}$ without irradiation<br>$IC_{50}$ with irradiation |
|---|---|---|---|
| Naphthazarin | 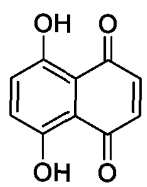 | 2219 ± 511<br>632 ± 175 (n=4) | 2476 ± 1506<br>353 ± 237 (n=5) |
| BJ-18 | 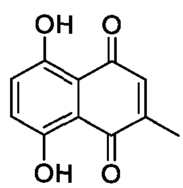 | 10933 ± 4079<br>2337 ± 846 (n=3) | -<br>87.6 ± 65.4 (n=2) |

(continued)

| name | structure | Ca [nM]<br>$IC_{50}$ without irradiation<br>$IC_{50}$ with irradiation | Tru [nM]<br>$IC_{50}$ without irradiation<br>$IC_{50}$ with irradiation |
|---|---|---|---|
| BJ-34 | 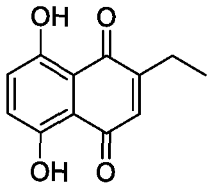 | -<br>715 ± 209 (n=3) | -<br>24.2 ± 20.6 (n=4) |
| BJ-35 | 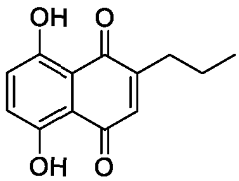 | -<br>599 ± 289 (n=3) | -<br>13.0 ± 12.2 (n=3) |
| BJ-36 | 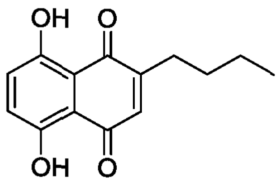 | -<br>556 ± 355 (n=3) | -<br>1.0 ± 0.3 (n=3) |
| DOS | 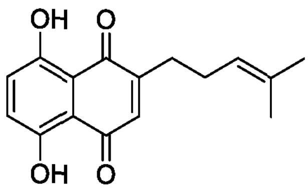 | -<br>213 ± 55 (n=5) | -<br>32.2 ± 17.4 (n=5) |
| BJ-28 | 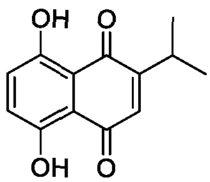 | -<br>1042 ± 185 (n=2) | -<br>42.7 (n=1) |
| BJ-19 | 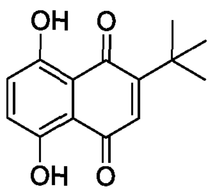 | -<br>7646 ± 4479 (n=2) | -<br>244 ± 192 (n=3) |
| BJ-27 | 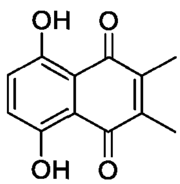 | -<br>2746 ± 70 (n=2) | -<br>321 ± 212 (n=2) |
| BJ-39 | 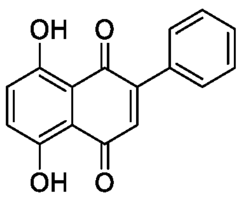 | nd | -<br>2.9 (n=1) |

(continued)

| name | structure | Ca [nM]<br>$IC_{50}$ without irradiation<br>$IC_{50}$ with irradiation | Tru [nM]<br>$IC_{50}$ without irradiation<br>$IC_{50}$ with irradiation |
|---|---|---|---|
| BJ-49 | 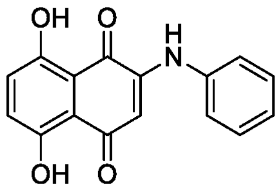 | 570 (n=1) | -<br>352 ± 150 (n=2) |
| BJ-SK-Amin | 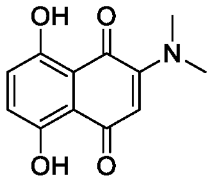 | -<br>1484 (n=1) | 8142<br>3338 (n=1) |
| Shikonin/ Alkannin | 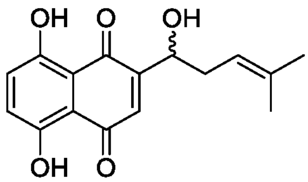 | -<br>1484 (n=1) | 10510 ± 6917<br>781 ± 407 (n=3) |
| Clotrimazol | Comparative compound for Tru | nd | 114 ± 61<br>109 ± 51 (n=10) |
| Nystatin | Comparative compound for Ca | 692 ± 128<br>1665 ± 886 (n=7) | nd |

wherein the curve fit was determined using Graphpad Prism 7 by the equation log(inhibitor) vs. response - variable slope, and

$$Y=Bottom + (Top-Bottom)/(1+10^{\wedge}((LogIC50-X)*HillSlope)),$$

with Top and Bottom determined as the plateaus of the Y-axis values, HillSlope describing the steepness of curves, with HillSlope = -1 as a suitable standard,

X = Log of active compound concentration, Y = value of response.

Ca = antimycotic activity against Candida albicans

Tru = antimycotic activity against Trichophyton rubrum

nd = not determined

\- = no fit possible

For comparison, 2-hydroxy-1,4-naphthochinone, which is a component of henna dye, was tested, and showed essentially no antimycotic effect upon irradiation.

The invention is now described in greater detail by way of examples and with reference to the figures which show in

- Fig. 1A and 1B the inhibition of C. albicans and of T. rubrum by exemplary pharmaceutical compounds,

- Fig. 2 a micrograph of a cross-section of a keratin plate with indication of measurement depth,
- Fig. 3A, 3B, 3C and 3D analyses of the migration of compounds, namely of comparative compound Amorolfin in Fig. 3A, of comparative compound Ciclopirox in Fig. 3B, and of Deoxyshikonin according to the invention in Fig. 3C, and in Fig. 3D analysis of migration of compound BJ-36 according to the invention,
- Fig. 4A, 4B analyses of migration of comparative compounds, in Fig. 4A of 5-amino laevulinic acid and in Fig. 4B of methylene blue.

Example 1: Anti-mycotic activity assay in pathogen suspension

For determining the $IC_{50}$ of the anti-mycotic activity of compounds, the human pathogen Candida albicans, strain DSM 1386, or clinical isolates of Trichophyton rubrum obtained from patient samples were used.

Candida albicans DSM 1386 was cultivated overnight in medium M186/3 (3.3 % glucose, 0.16 % peptone from soybeans, 0.1% yeast extract, 0.1 % malt extract). Test samples were added in triplicates to a 96-well plate for a final assay concentration of 10 μM and 100 μM of pharmaceutical compounds. 200 μl of the yeast was added to each well after diluting the overnight culture to an optical density (600 nm) of 0.03. The wells were irradiated using a custom-made light source with a peak wavelength of 519 nm (8 LEDs, 4600 mW, 6.5 $mW/cm^2$, Fa. Sahlmann Photochemical Solutions, Bad Segeberg, Germany). Handling of the test compounds and well plates took place in a darkened room equipped with red-light LEDs to protect them from green light. Microplates were incubated for 5 h at 28 °C on a shaker at 200 rpm. Subsequently, 10 μl resazurin solution (0.3 mg/ml in phosphate buffer) was added to each well and the microplates were incubated again for 30 min before measuring fluorescence (560/590 nm) using a microplate reader (Tecan Infinite M200, Tecan, Männedorf, Switzerland). Nystatin was used as positive control. For $IC_{50}$ determination a dilution series of the test samples was prepared and $IC_{50}$ value was calculated by Graph Pad Prism 7 as the concentration that show 50% inhibition of growing.

Trichophyton rubrum I/95 (patient isolate) was cultivated for 1 week on GPY (0.1% glucose, 0.05% petone, 0.01 yeast extract, 1.5% agar) at 28°C. A suspension of $5x10^4$ spores/ml in M186 (1% glucose, 0.5% peptone from soybeans, 0.3% yeast extract, 0.3% malt extract) was prepared and added to each well of a microplate containing one of the pharmaceutical compounds, which were pipetted in triplicates for a final concentration of 10 μm and 100 μM. The wells were irradiated using the custom-made light source with a peak wavelength of 519 nm. Handling of the test compounds and well plates took place in a darkened room equipped with a red-light LED to protect them from green light. After incubation for 72 h at 28 °C and 120 rpm the optical density at 600 nm was measured using the microplate reader Tecan Infinite M200. The resulting values were compared with a positive control (10 μM Clotrimazol) and a negative control (no compound) on the same plate. For $IC_{50}$ determination a dilution series of the test samples was prepared and $IC_{50}$ value was calculated by Graph Pad Prism 7 as the concentration that show 50 % inhibition of growing.

Fig. 1A shows the $IC_{50}$ values for deoxyshikonin against Candida albicans strain and Fig. 1B against the T. rubrum patient isolate, both in the absence of activating irradiation (lower curves, dark) and in the presence of activating irradiation (519 nm, upper curves). The $IC_{50}$ under irradiation against C. albicans was determined to 213.3 $\pm$ 54.7 nM, against T. rubrum to 32.2 $\pm$ 17.4 nM. For the comparative compound Nystatin an $IC_{50}$ = 692.4 $\pm$ 140.4 nM without irradiation, and a lower activity under irradiation, which is believed to be caused by decomposition of Nystatin under irradiation.

The results are summarized in the above table, showing that the pharmaceutical compounds of the invention under irradiation are effective against the pathogens causing onychomycoses. Further, the results show that the activity of the pharmaceutical compounds is significantly lower in the absence of irradiation, which allows application of the compounds in the absence of activating irradiation without generating ROS that might damage soft tissue.

Example 2: Permeation of pharmaceutical compounds into nail

For testing the permeation of pharmaceutical compounds into nail, a Franz diffusion cell (obtained from Permegear, Germany) was used, in which a bovine hoof plate, 2 mm thickness, is arranged to separate a first volume of phosphate buffered saline pH = 8.1 with 42 % vol./vol. ethanol containing a pharmaceutical compound from a second volume of the same fluid but without pharmaceutical compound, wherein the second volume is circulated for 20 h at 20 °C and then analysed for presence of the pharmaceutical compound. It was found that the compounds of the invention migrated from the first volume into the hoof plate, and the compounds could not be detected in the second volume. This indicates that the pharmaceutical compounds migrate into the keratin plate that was represented by the hoof plate, and that the compounds essentially did not diffuse out of the keratin plate into the second volume. This shows that the compounds of the invention permeate into the keratin and do not diffuse out of the keratin, indicating that nail matrix adjacent to the keratin does not receive the compounds, and therefore the nail matrix is not directly affected by activity of the pharmaceutical compounds under irradiation.

As a further analysis, a bovine hoof plate, 2 mm thickness, was arranged on water-saturated cotton gauze, and

pharmaceutical compound was deposited on the opposite surface of the hoof plate. This arrangement was incubated under static conditions at room temperature for 5 days. The pharmaceutical compound was used at 5 wt.-% in a preliminary formulation of 12.5 wt.-% Eudragit RL PO, 60 wt.-% ethanol, 15 wt.-% ethyl acetate, 5 wt.-% butyl acetate, 2.5 wt.-% triacetin, which formulation should mimic the Loceryl lacquer. For comparison, the topic antimycotics amorolfin in Loceryl lacquer and ciclopirox in Ciclopoli were used in parallel.

After the incubation for 5 d, the lacquer compositions were carefully removed and cross-sections from the hoof plates were made. Fig. 2 shows a microscopic picture of a hoof plate cross-section, with an indication of measurement points X numbered 1.0, 1.1, 1.2 close to the surface, and measurement points X numbered 2.0, 2.1, 2.2 at a depth of 10 μm. This pattern of measurement points, each at a depth greater by 10 μm, up to a depth of at least 100 μm, was used for Raman spectroscopy for presence of pharmaceutical compound.

Fig. 3A for amorolfin, Fig. 3B for ciclopirox, Fig. 3C for deoxyshikonin, and Fig. 3D as representatives of the compounds of the invention, show the concentration of the respective compound in the different depths of the hoof plate, wherein increasing depths showed lower compound concentrations. Raman spectroscopy showed that the comparative amorolfin could not be detected inside the keratin cross-section as only the keratin baseline was detected. For ciclopirox, migration up to a depth of 40 μm into the keratin cross-section was found. Deoxyshikonin was found to migrate to a depth of more than 50 μm into the keratin cross-section, compound BJ-36 was found to migrated to a depth of more than 70 μm into the keratin cross-section.

For comparison, 5-amino laevulinic acid and methylene blue were applied in the same basic lacquer as above. Raman spectroscopy of the hoof plate showed a migration to a depth of 30 μm for 5-amino laevulinic acid (Fig. 4A), and to a depth of 50 μm for methylene blue (Fig. 4B).

On the exemplary pharmaceutical compounds of the invention, these results show that the compounds of the invention show good penetration into nails and hooves, even when using it in the preliminary lacquer formulation that was not optimized, with only one topical application.

Example 3: Anti-mycotic activity assay from nail

For testing the effect of the pharmaceutical compounds in a nail, compounds were applied as a solution of 5 mg/ml or 10 mg/ml in nitrocellulose-based cosmetic nail lacquer, diluted 1: 1 with ethyl acetate, onto cow horn membranes representing nail.

Trichophyton rubrum I/95 (patient isolates) was cultivated for 1-3 weeks on GPY (0.1 % glucose, 0.05 % peptone, 0.01 yeast extract, 1.5 % agar) at 28°C. For the infected nail model, petri dishes with M186 agar (1% glucose, 0.5% peptone from soybeans, 0.3% yeast extract, 0.3% malt extract, 1.5 % agar, supplemented with 100 U/ml penicillin and 10 0mg/ml streptomycin) where inoculated with the pathogen using sterile cotton swabs. Afterwards autoclaved cow horn membranes (in DPBS-buffer solution, 100 μm thick, 8 mm in diameter) where put onto the agar (5 per petri dish) followed by incubation for 48 h at 29 °C. As an example of the invention, Deoxyshikonin was used, and clotrimazol as control, the diluted nail laquer as vehicle control. The formulations were applied to 15 μL/membrane to the cow horn membranes using an Eppendorf pipette. After incubation for another 24 h four membranes of each type (control, vehicle, 5 mg/ml or 10 mg/ml deoxyshikonin) were transferred onto fresh petri dishes with the surface to which the formulation was applied now facing the agar. The dishes were irradiated two times for 5 min each with a peak wavelength of 519 nm (8 LEDs, 4600 mW, 6.5 mW/cm$^2$). Afterwards formulations were applied (15 μL/membrane) onto the untreated side of the membranes (facing upwards), they were allowed to dry and were irradiated again for 5 min (2x). The dishes were incubated for 24 h at 29 °C and irradiated again for 5 min (2x). After 7 days of incubation at 29 °C the dishes were evaluated. Anytime handling the compounds and the agar plates after application of the formulations the ambient light was turned off and a red-light LED was used to protect them from any activating light source.

It was found that with irradiation the deoxyshikonin from the cow horn membranes effectively suppressed growth of T. rubrum on and in the horn membranes as well as in the surrounding medium, whereas without irradiation, growth of T. rubrum was reduced compared to both controls but not suppressed.

The same anti-mycotic activity assay was performed with compound BJ-36, showing that the compound of the invention after irradiation at 519 nm effectively eliminated any growth of T. rubrum and of C. albicans in the cow horn membrane, without diffusion of the compound into the supporting agar. Without irradiation, compound BJ-36 was not effective in suppressing growth of the infectious fungi. Comparative testing of methylene blue and of 5-amino laevulinic acid showed that these compounds did not suppress growth of the infectious fungi, with or without irradiation, and further showed diffusion of methylene blue into the supporting agar, indicating that this compound also affects soft tissue surrounding the nail.

This result shows that the compounds of the invention are effective in the treatment of onychomycoses in photodynamic treatment using irradiation of the nail to which the compound has been applied, wherein the irradiation has a wavelength for which nail is permeable and which wavelength activates the compound. A preferred wavelength is about 519 nm.

## Claims

1. Pharmaceutical compound for use in the treatment of onychomycoses, the treatment comprising irradiation of the nail or hoof at a wavelength of 450 to 700 nm, **characterized in that** the compound comprises a structure selected from

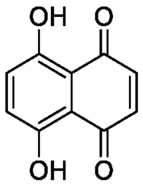
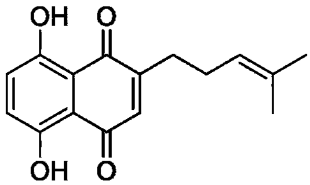
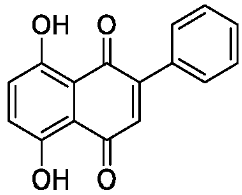
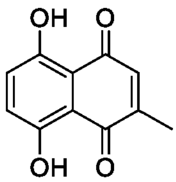
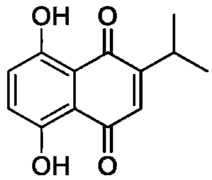
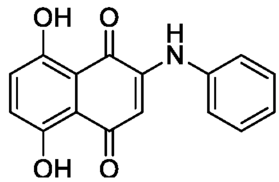
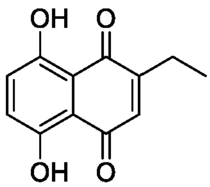
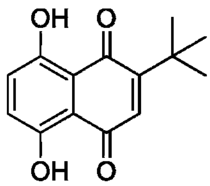
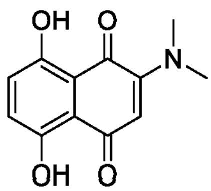
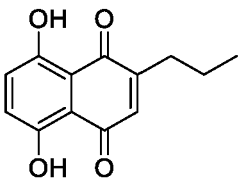
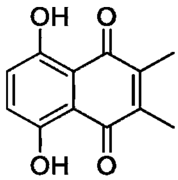
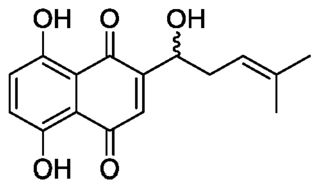
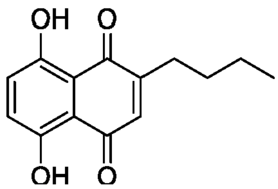

2. Pharmaceutical compound for use in the treatment of onychomycoses according to claim 1, wherein the treatment comprises irradiation of the compound at a wavelength of 500 to 600 nm subsequent to topical application of the compound to a nail or hoof.

3. Pharmaceutical compound for use in the treatment of onychomycoses according to one of the preceding claims, wherein the treatment comprises penetration of the compound into nail or hoof, without penetration of the compound from the nail into underlying nail matrix.

4. Pharmaceutical compound for use in the treatment of onychomycoses according to one of the preceding claims, wherein the onychomycoses is caused by Trichophyton rubrum and/or Candida albicans.

5. Pharmaceutical compound for use in the treatment of onychomycoses according to one of the preceding claims, wherein the onychomycosis comprises presence of fungal spores.

6. Pharmaceutical compound for use in the treatment of onychomycoses according to one of the preceding claims, wherein the treatment comprises application of the compound to a nail or hoof in the absence of light of a wavelength of

500 to 600 nm.

7. Pharmaceutical compound for use in the treatment of onychomycoses according to one of the preceding claims, wherein the treatment subsequent to application of the compound to a nail or hoof and prior to irradiation of the compound comprises removing the compound from skin.

**Patentansprüche**

1. Pharmazeutische Verbindung zur Verwendung bei der Behandlung von Onychomykosen, wobei die Behandlung die Bestrahlung des Nagels oder Hufes mit einer Wellenlänge von 450 bis 700 nm umfasst, **dadurch gekennzeichnet, dass** die Verbindung eine Struktur umfasst, die ausgewählt ist aus

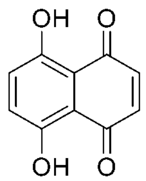
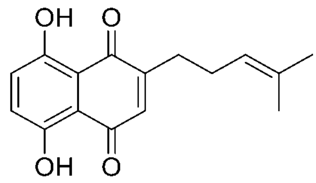
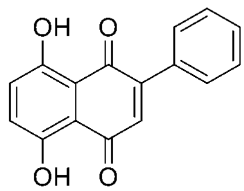
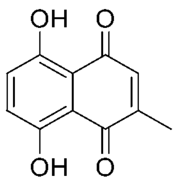
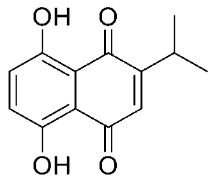
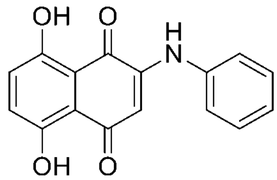
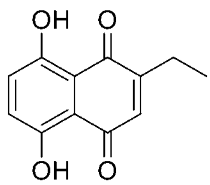
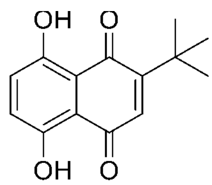
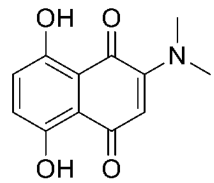
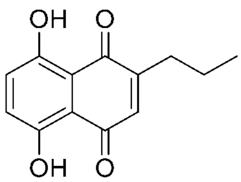
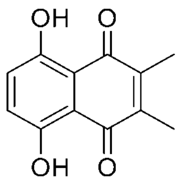
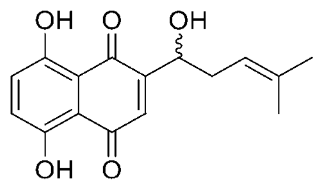
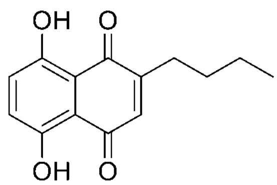

2. Pharmazeutische Verbindung zur Verwendung bei der Behandlung von Onychomykosen gemäß Anspruch 1, wobei die Behandlung die Bestrahlung der Verbindung mit einer Wellenlänge von 500 bis 600 nm nach topischer Anwendung der Verbindung auf einen Nagel oder Huf umfasst.

3. Pharmazeutische Verbindung zur Verwendung bei der Behandlung von Onychomykosen gemäß einem der vorstehenden Ansprüche, wobei die Behandlung das Eindringen der Verbindung in den Nagel oder Huf umfasst, ohne dass die Verbindung vom Nagel in die darunterliegende Nagelmatrix eindringt.

4. Pharmazeutische Verbindung zur Verwendung bei der Behandlung von Onychomykosen gemäß einem der vorstehenden Ansprüche, wobei die Onychomykose durch Trichophyton rubrum und/oder Candida albicans verursacht wird.

**5.** Pharmazeutische Verbindung zur Verwendung bei der Behandlung von Onychomykosen gemäß einem der vorstehenden Ansprüche, wobei die Onychomykose das Vorhandensein von Pilzsporen umfasst.

**6.** Pharmazeutische Zusammensetzung zur Verwendung bei der Behandlung von Onychomykosen gemäß einem der vorstehenden Ansprüche, wobei die Behandlung das Auftragen der Zusammensetzung auf einen Nagel oder Huf in Abwesenheit von Licht einer Wellenlänge von 500 bis 600 nm umfasst.

**7.** Pharmazeutische Verbindung zur Verwendung bei der Behandlung von Onychomykosen gemäß einem der vorstehenden Ansprüche, wobei die Behandlung nach dem Auftragen der Verbindung auf einen Nagel oder Huf und vor der Bestrahlung der Verbindung das Entfernen der Verbindung von der Haut umfasst.

## Revendications

**1.** Composé pharmaceutique destiné à être utilisé dans le traitement des onychomycoses, ledit traitement comprenant l'irradiation de l'ongle ou du sabot à une longueur d'onde comprise entre 450 et 700 nm, **caractérisé en ce que** le composé comprend une structure choisie parmi

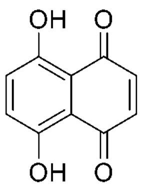
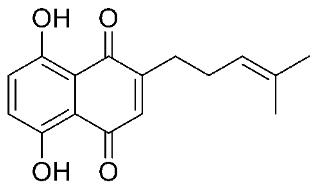
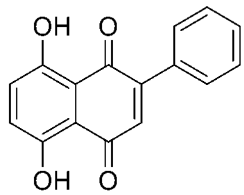
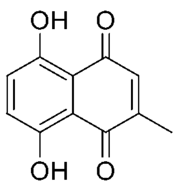
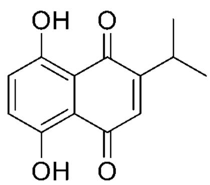
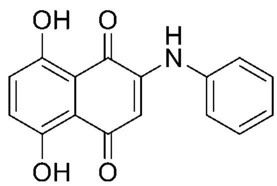
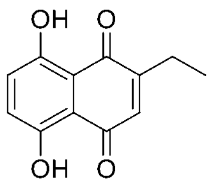
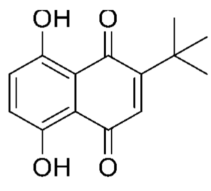
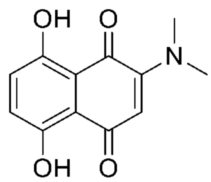
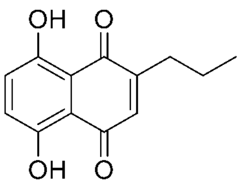
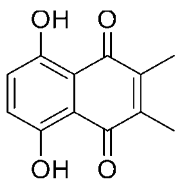
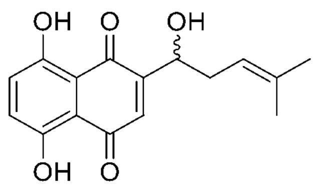
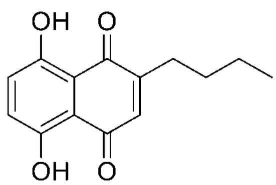

2. Composé pharmaceutique destiné au traitement des onychomycoses selon la revendication 1, dans lequel le traitement comprend l'irradiation du composé à une longueur d'onde comprise entre 500 et 600 nm après application topique du composé sur un ongle ou un sabot.

3. Composé pharmaceutique destiné au traitement des onychomycoses selon l'une des revendications précédentes, dans lequel le traitement comprend la pénétration du composé dans l'ongle ou le sabot, sans pénétration du composé depuis l'ongle dans la matrice unguéale sous-jacente.

4. Composé pharmaceutique destiné à être utilisé dans le traitement des onychomycoses selon l'une des revendications précédentes, dans lequel l'onychomycose est causée par Trichophyton rubrum et/ou Candida albicans.

5. Composé pharmaceutique destiné au traitement des onychomycoses selon l'une des revendications précédentes, dans lequel l'onychomycose comprend la présence de spores fongiques.

6. Composé pharmaceutique destiné au traitement des onychomycoses selon l'une des revendications précédentes, dans lequel le traitement comprend l'application du composé sur un ongle ou un sabot en l'absence de lumière d'une longueur d'onde comprise entre 500 et 600 nm.

7. Composé pharmaceutique destiné à être utilisé dans le traitement des onychomycoses selon l'une des revendications précédentes, dans lequel le traitement, après l'application du composé sur un ongle ou un sabot et avant l'irradiation du composé, comprend l'élimination du composé de la peau.

Fig. 1A

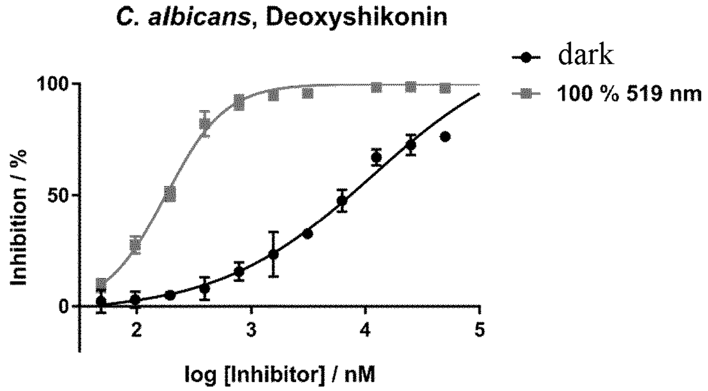


Fig. 1B

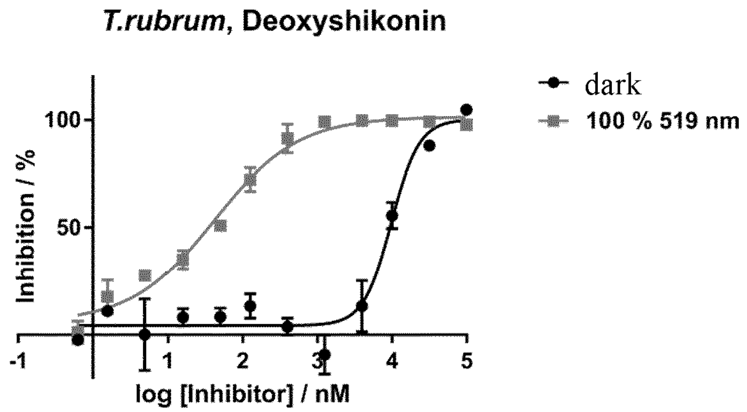


Fig. 2

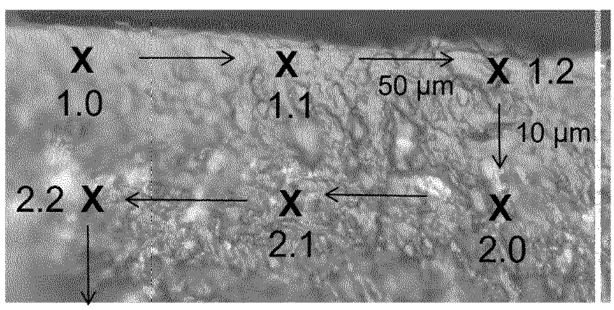

Fig. 3A

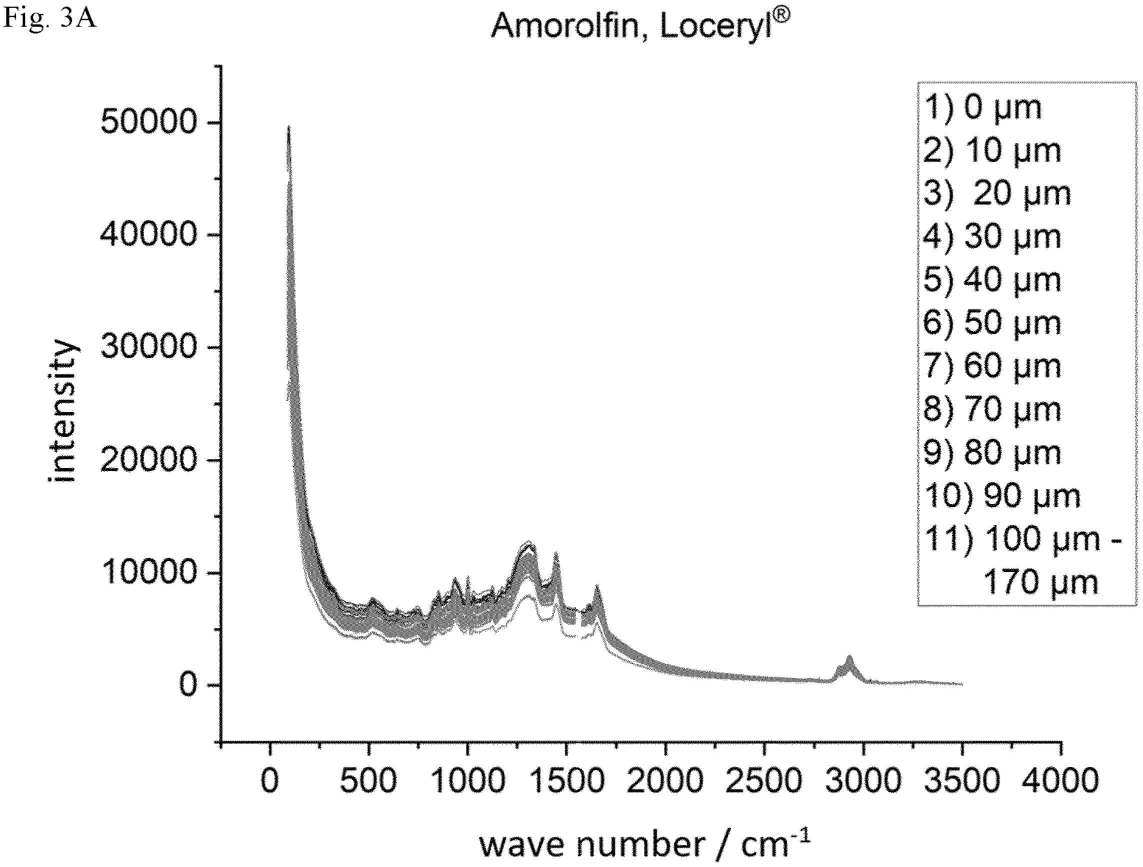
Amorolfin, Loceryl®
intensity
wave number / cm-1
50000
40000
30000
20000
10000
0
0
500
1000
1500
2000
2500
3000
3500
4000
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm

Fig. 3B

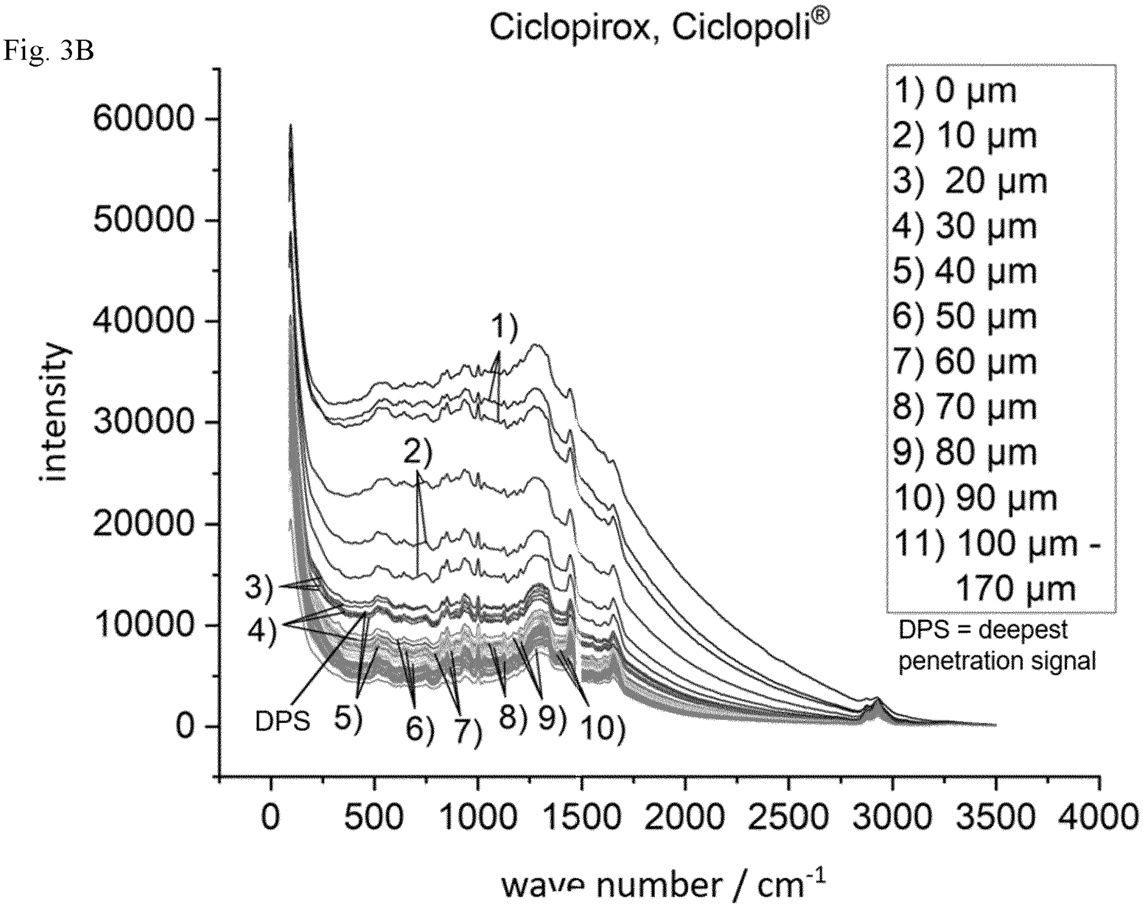
Ciclopirox, Ciclopoli®
intensity
wave number / cm-1
60000
50000
40000
30000
20000
10000
0
0
500
1000
1500
2000
2500
3000
3500
4000
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm
DPS = deepest penetration signal
1)
2)
3)
4)
DPS
5)
6)
7)
8)
9)
10)

Fig. 3C

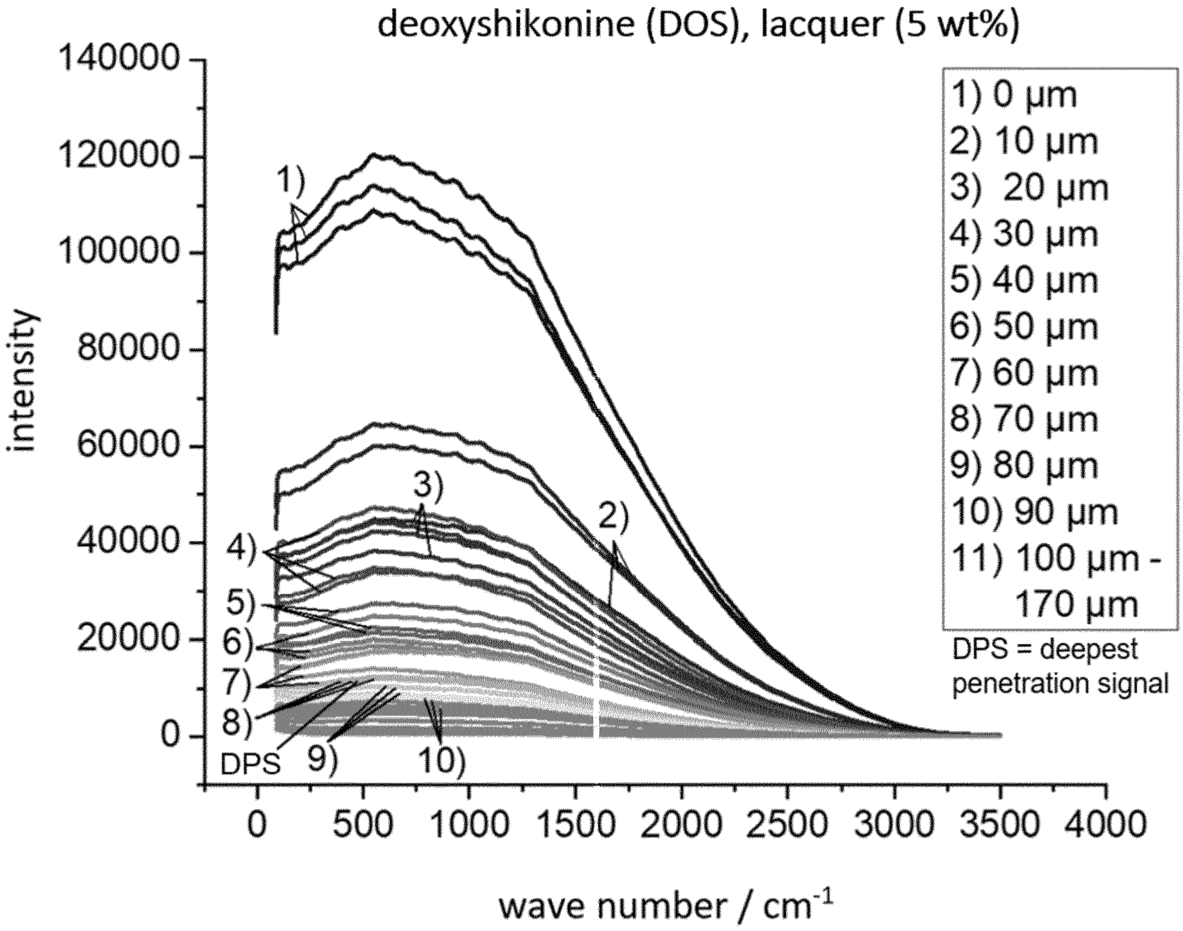
deoxyshikonine (DOS), lacquer (5 wt%)
intensity
140000
120000
100000
80000
60000
40000
20000
0
0
500
1000
1500
2000
2500
3000
3500
4000
wave number / cm-1
1) 0 µm
2) 10 µm
3) 20 µm
4) 30 µm
5) 40 µm
6) 50 µm
7) 60 µm
8) 70 µm
9) 80 µm
10) 90 µm
11) 100 µm - 170 µm
DPS = deepest penetration signal
1)
2)
3)
4)
5)
6)
7)
8)
DPS
9)
10)

Fig. 3D

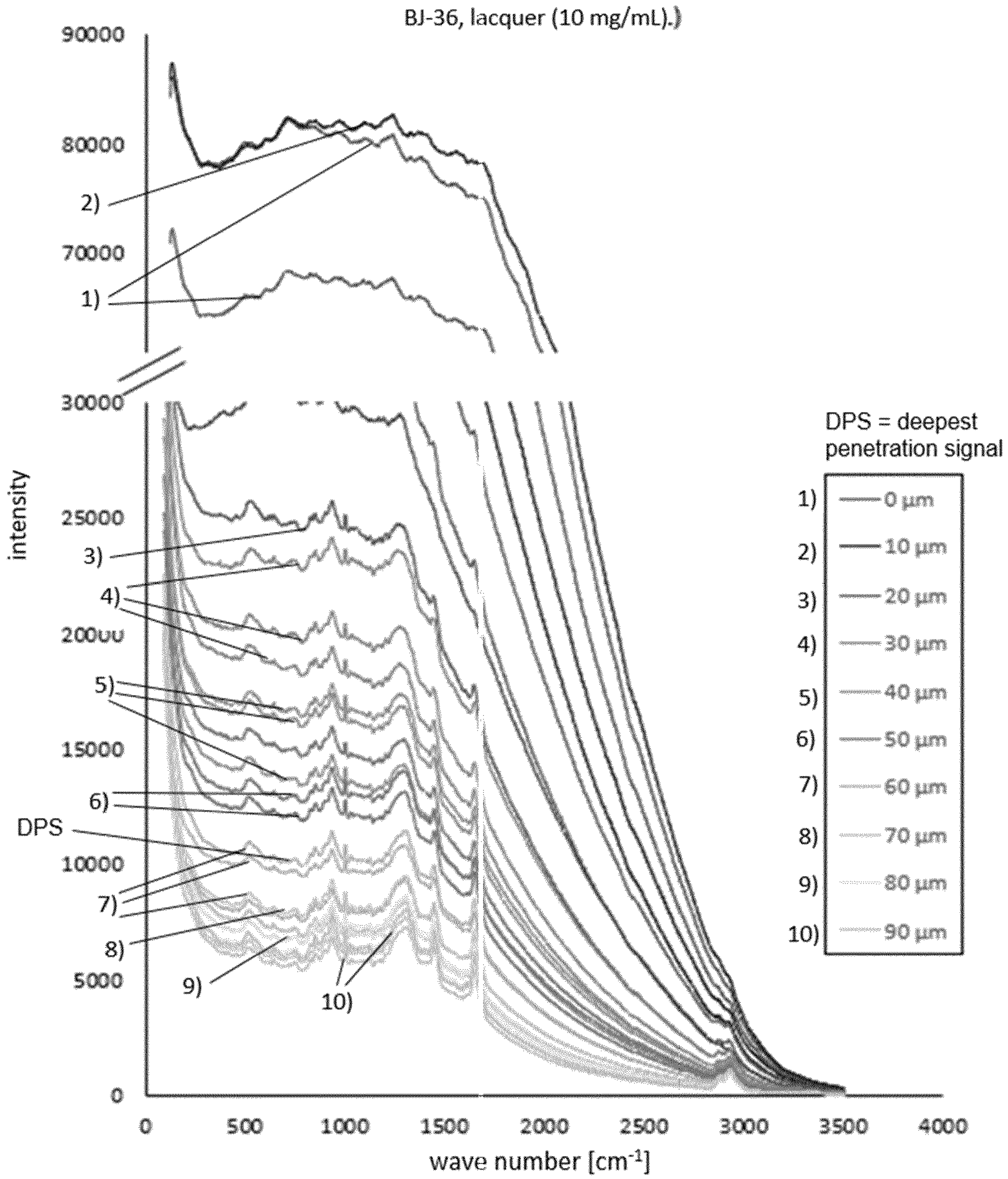
BJ-36, lacquer (10 mg/mL).)
intensity
90000
80000
70000
30000
25000
20000
15000
10000
5000
0
2)
1)
3)
4)
5)
6)
DPS
7)
8)
9)
10)
0
500
1000
1500
2000
2500
3000
3500
4000
wave number [cm-1]
DPS = deepest penetration signal
1) 0 µm
2) 10 µm
3) 20 µm
4) 30 µm
5) 40 µm
6) 50 µm
7) 60 µm
8) 70 µm
9) 80 µm
10) 90 µm

Fig. 4A

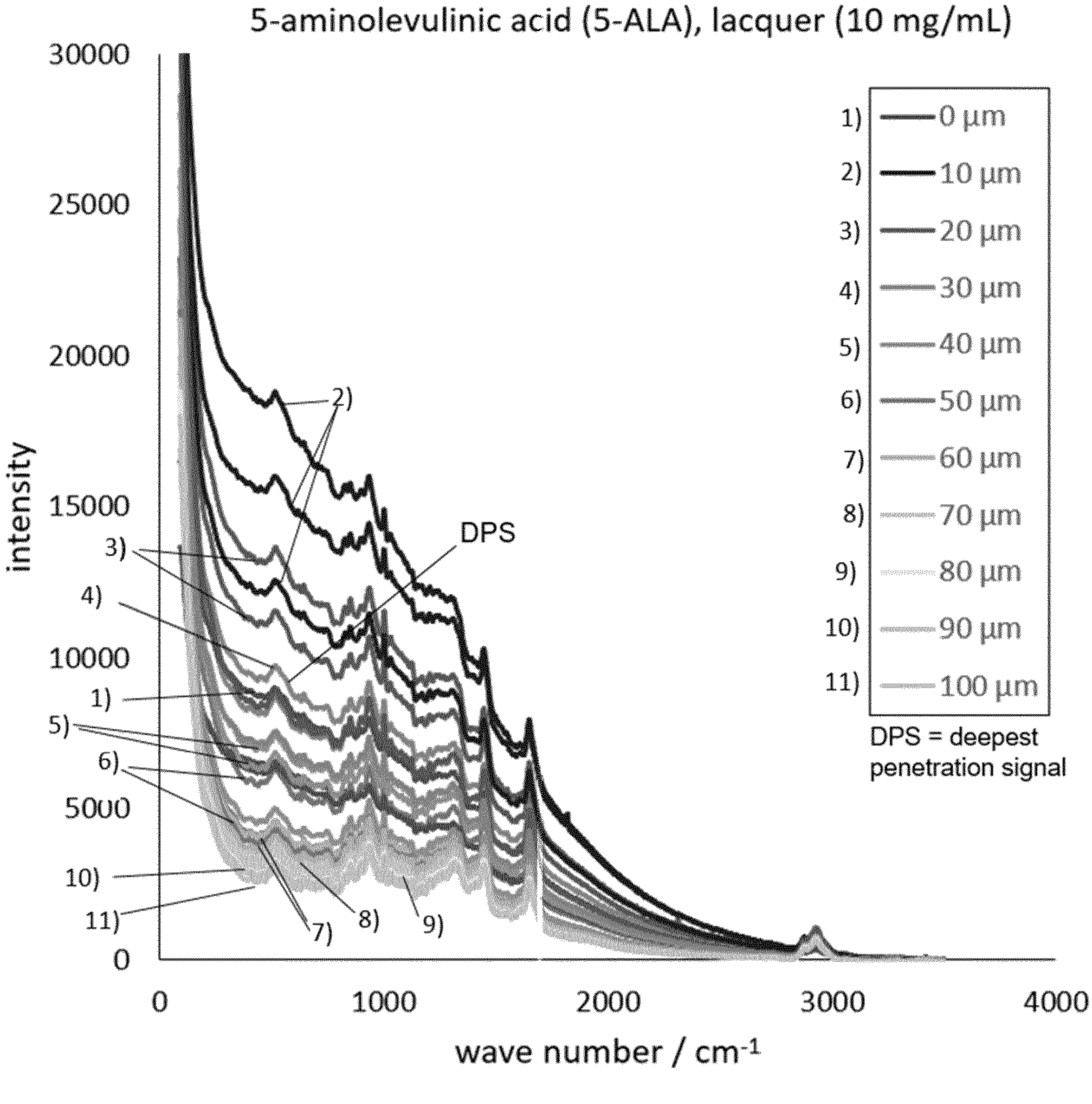
5-aminolevulinic acid (5-ALA), lacquer (10 mg/mL)
intensity
30000
25000
20000
15000
10000
5000
0
0
1000
2000
3000
4000
wave number / cm-1
1) 0 µm
2) 10 µm
3) 20 µm
4) 30 µm
5) 40 µm
6) 50 µm
7) 60 µm
8) 70 µm
9) 80 µm
10) 90 µm
11) 100 µm
DPS = deepest penetration signal
DPS
1)
2)
3)
4)
5)
6)
7)
8)
9)
10)
11)

Fig. 4B

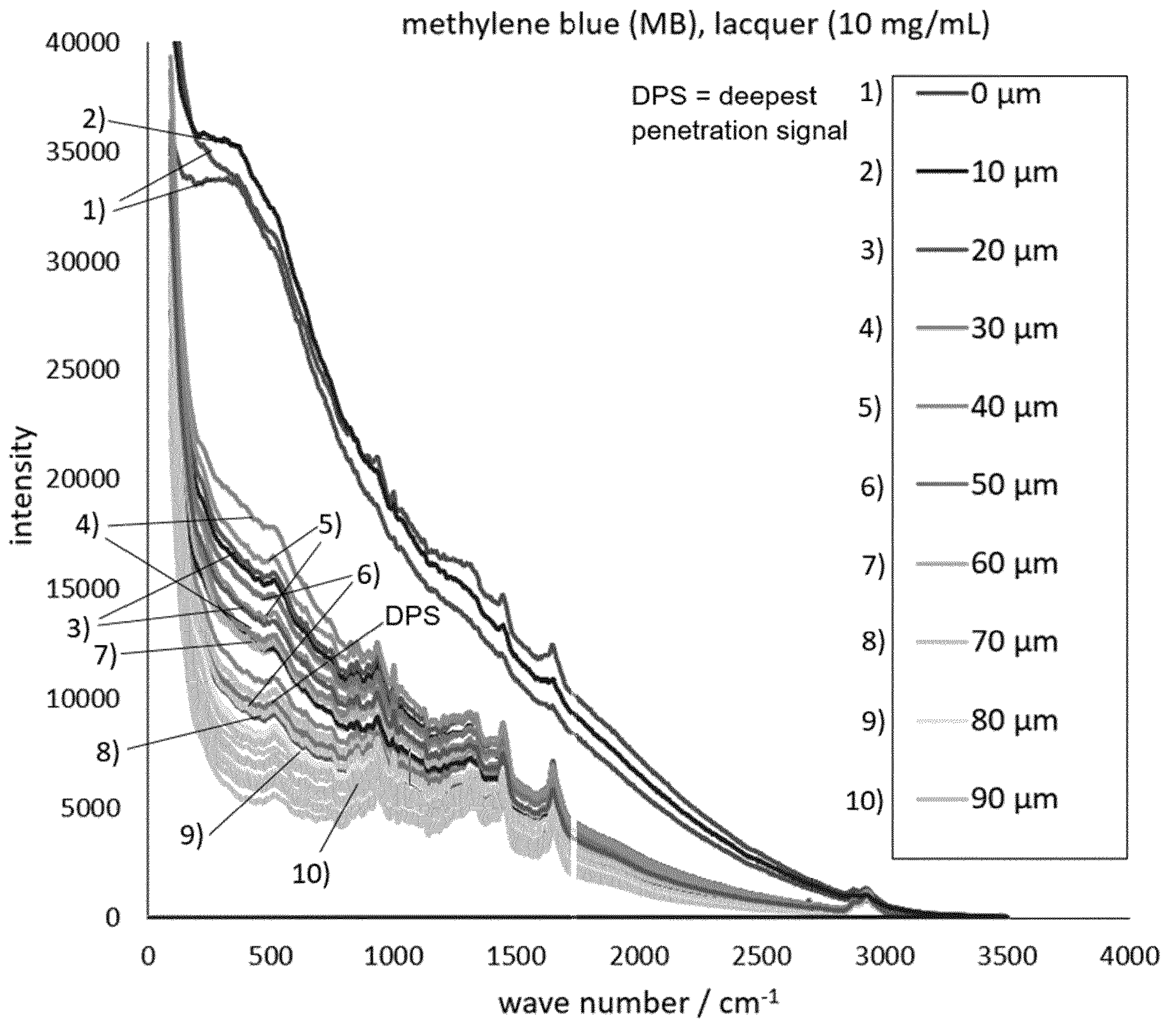
methylene blue (MB), lacquer (10 mg/mL)
DPS = deepest penetration signal
1) 0 µm
2) 10 µm
3) 20 µm
4) 30 µm
5) 40 µm
6) 50 µm
7) 60 µm
8) 70 µm
9) 80 µm
10) 90 µm
intensity
40000
35000
30000
25000
20000
15000
10000
5000
0
2)
1)
4)
5)
6)
3)
7)
DPS
8)
9)
10)
0
500
1000
1500
2000
2500
3000
3500
4000
wave number / cm-1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 200219 B1


### Non-patent literature cited in the description


- **PAPAGEORGIOU et al.** *Angew. Chem Int. Ed.*, 1999, 270-300
- **MENEZES et al.** *Journal of Photochemistry & Photobiology B*, 2016, 1-12
- **GUPTA et al.** *JEADV*, 2020, 1972-1990